**Europäisches Patentamt**

**European Patent Office**

**Office européen des brevets**

(19)

(11) Veröffentlichungsnummer: **0 316 371 B1**

(12) # EUROPÄISCHE PATENTSCHRIFT

(45) Veröffentlichungstag der Patentschrift:
30.10.91 Patentblatt 91/44

(51) Int. Cl.[5]: **A61F 5/01**, A61B 17/60

(21) Anmeldenummer: **88902804.9**

(22) Anmeldetag: **31.03.88**

(86) Internationale Anmeldenummer:
**PCT/DE88/00208**

(87) Internationale Veröffentlichungsnummer:
**WO 88/07357 06.10.88 Gazette 88/22**

(54) **VORRICHTUNG ZUM EINRICHTEN EINER WIRBELSÄULE.**

(30) Priorität: **02.04.87 DE 3711091**

(43) Veröffentlichungstag der Anmeldung:
**24.05.89 Patentblatt 89/21**

(45) Bekanntmachung des Hinweises auf die
Patenterteilung:
**30.10.91 Patentblatt 91/44**

(84) Benannte Vertragsstaaten:
**CH FR GB IT LI NL**

(56) Entgegenhaltungen:
**EP-A- 0 159 007**
**GB-A-21 571 79**
**US-A-46 205 33**

(73) Patentinhaber: **Kluger, Patrick, Dr. med.**
**Heinrich-Hammer-Strasse 12**
**W-7904 Erbach (DE)**

(72) Erfinder: **Kluger, Patrick, Dr. med.**
**Heinrich-Hammer-Strasse 12**
**W-7904 Erbach (DE)**

(74) Vertreter: **Walther, Horst, Dipl.-Ing.**
**Wilhelmshöher Allee 275 Postfach 41 01 08**
**W-3500 Kassel (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

Die Erfindung betrifft eine Vorrichtung zum Einrichten einer Wirbelsäule mit geschädigten Wirbelkörpern, bestehend aus einem auf einer Führungsachse verstellbaren Arm und einem darauf angeordneten festen Arm, wobei jeder Arm einen in zwei Ebenen verstellbaren Aufnahmehohlkörper für eine Verlängerungsstange trägt, die mit einer Knochenschraube verbindbar ist.

Eine derartige Vorrichtung ist aus der EP-A-0159007 bekannt. Bei der bekannten Vorrichtung sind die auf der Führungsachse angeordneten Arme räumlich gekrümmt tragen und endseitig gelenkig angebrachte Gewindezapfen, auf denen eine Aufnahmehülse drehbar und mit Hilfe einer Flügelmutter feststellbar ist.

Die Ausführung der Einrichtung in dieser Art, insbesondere die Anordnung der Aufnahmehülse und deren Befestigung, entspricht jedoch nicht den Anforderungen, die ein Operateur, insbesondere im Hinblick auf eine leichte Handhabbarkeit, an ein solches Gerät stellen muß.

Der Erfindung liegt daher die Aufgabe zugrunde, die Vorrichtung zur Einrichtung einer Wirbelsäule mit geschädigten Wirbelkörpern so zu verbessern, daß eine leichtere Handhabung erreicht ist.

Nach der Erfindung wird das dadurch erreicht, daß jeder Arm ein Zwischengelenkstück mit zwei Gelenken trägt, wobei am Zwischengelenkstück ein Träger für den Aufnahmehohlkörper angelenkt ist, der als fixierbares Zylinderstück mit einer Bohrung ausgebildet ist, die im radialen Abstand u.senkrecht zur Zylinderstück-Längsachsenebene angeordnet ist. Dabei ist als Ausgestaltung der Erfindung spitzwinklig zur Bohrung eine Fixierschraube zur Fixierung des Verlängerungsstabes vorgesehen. Das Zylinderstück ist in weiterer Ausgestaltung axial verstellbar und in einer Endlage feststellbar.

Gemäß einer besonders vorteilhaften Ausführungsform trägt zu diesem Zweck der Träger einen senkrechten Gewindezapfen, der an einem Ende eine Rasterplatte und in Abstand davon eine auf dem Gewinde drehbare Mutter aufweist, wobei zwischen der Mutter und der Rasterplatte der Aufnahmehohlkörper drehbar und axial verstellbar angebracht ist.

Durch diese Gestaltung kann die Verlängerungsstange leichter und zugänglicher in den Aufnahmehohlkörper eingebracht und fixiert werden, indem das Zylinderstück mittels der Mutter axial verschoben wird, wobei die Rasterplatte erfaßt wird, so daß einerseits die Rasten der Rasterplatte als auch andererseits die Rasten des Zylinderstückes in Eingriff sind. Hinzu kommt, daß die Fixierschraube zur Feststellung der Verlängerungsstange durch ihre schräge Lage vom Operateur leichter bedienbar ist. Darüber hinaus kann die gesamte Vorrichtung durch die Anordnung der Zwischengelenkstücke leichter eingerichtet werden.

Eine weitere Verbesserung der Handhabung der Vorrichtung wird dadurch erreicht, daß die Verlängerungsstange endseitig eine wannenförmige Ausbildung für die Aufnahme der Knochenschraube aufweist, die kopfseitig ein entsprechend gestaltetes Profilstück mit Rasterung für die Aufnahme eines Fixierstabes besitzt. Die wannenförmige Ausbildung weist dabei mehr als Halbkreisform auf. so daß die Verlängerungsstange nur axial herausziehbar ist.

Dadurch ist auf einfache Weise das Aufsetzen des Verlängerungsstabes auf die Knochenschraube ermöglicht, weil lediglich durch Aufschieben des wannenförmigen Teiles auf das entsprechende Profilstück die Verbindung ermöglicht wird.

Auch dabei erfolgt die Befestigung der Knochenschraube in der wannenförmigen Ausbildung durch eine Schraube, die spitzwinklig zur Achse der Verlängerungsstange einschraubbar ist, so daß auch hier der Operateur das Gerät leichter handhaben kann.

Eine andere Ausführungsform der Verbindung der Knochenschraube mit der Verlängerungsstange kann darin bestehen, daß an der Knochenschraube ein zylinderförmiges Walzenstück angeordnet ist, das stirnseitig eine Rasterung für die Aufnahme eines Fixierstabes aufweist, wobei dann die Verlängerungsstange endseitig ein der Zylinderform entsprechendes Gabelstück besitzt, damit diese Verlängerungsstange auf das Walzenstück aufschiebbar ist. Dabei ist die Verlängerungsstange mit dem Walzenstück drehfest verbunden. beispielsweise dadurch, daß an dem Walzenstück ein Aufsatz angebracht ist, der in eine entsprechende Aussparung in dem Gabelstück der Verlängerungsstange eingreift.

Eine besonders zweckmäßige Ausbildung des Fixierstabes besteht darin, daß der Fixierstab aus Teleskopstücken gebildet wird, dessen einschiebbarer Teil Fixierkerben aufweist, wobei auch dieser Fixierstab endseitig eine Rasterplatte mit Bohrung besitzt.

Dieser Fixierstab dient bekanntlich dazu, die mit Hilfe der Einrichtung eingerichteten Wirbelkörper in der eingerichteten Lage zu fixieren, so daß die geschädigte Wirbelstelle durch den Fixierstab überbrückt ist. Die Länge des Fixierstabes und auch die relative Lage der endseitigen Rasterplatten zueinander wird dann in der Weise fixiert, daß mit Hilfe einer Lochschablone das äußere Teleskopstück mit Hilfe einer entsprechend ausgebildeten Zange kerbförmig eingedrückt wird, wobei dann diese Kerbe genau in der Kerbe des einschiebbaren Teleskopstückes liegt.

Für kurze Überbrückungslängen ist ein aus einem Stück bestehender Fixierstab mit endseitiger Rasterplatte mit Bohrung vorgesehen.

In der Zeichnung ist eine beispielsweise Ausführungsform dargestellt.

Fig. 1 zeigt die Vorrichtung in Aufsicht;

Fig. 2 ist ein Schnitt gemäß der Linie II-II durch das Zylinderstück;

Fig. 3 ist ein Schnitt gemäß der Linie III-III durch die Verlängerungsstange;

Fig. 4 zeigt die Fixierung der Knochenschrauben mit Hilfe eines Fixierstabes;

Fig. 5 zeigt einen Schnitt gemäß der Linie V-V in Fig. 4;

Fig. 6 und 7 zeigen eine andere Ausbildung von Knochenschrauben und Verlängerungsstange.

Die Vorrichtung zum Einrichten einer Wirbelsäule besteht aus der Führungsachse 1 und dem daran fest angeordneten Arm 2 und dem mit Hilfe einer Gewindespindel 3 verschiebbar bzw. verstellbar angeordneten Arm 4. Jeder Arm 2 bzw. 4 trägt ein Zwischengelenkstück 5 bzw. 5a mit den Gelenkachsen 6 und 7. An dem Zwischengelenkstück 5 bzw. 5a ist ein Träger 8 bzw. 8a angelenkt, der den insgesamt mit 9 bezeichneten Aufnahmehohlkörper 9 trägt. Die Verstellebene des Aufnahmehohlkörpers steht senkrecht zu der Verstellebene des Trägers 8 bzw. 8a.

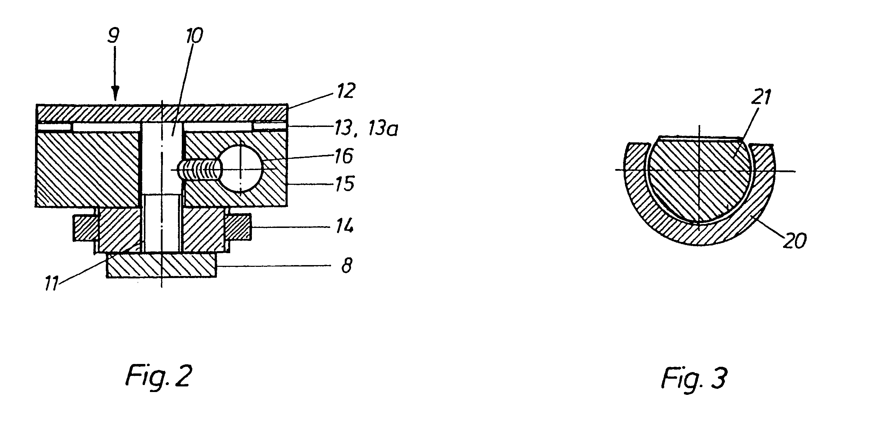

Die Ausbildung des Aufnahmehohlkörpers 9 ist in Fig. 2 im einzelnen dargestellt. Dabei ist an dem Arm 8 fest ein senkrechter Gewindezapfen 10 angeordnet, der im unteren Teil das Gewinde 11 trägt.

Am einen Ende des Gewindezapfens ist die Rasterplatte 12 fest angeordnet, welche die Rasten 13 besitzt. Am unteren Ende des Gewindezapfens, also auf dem Gewinde 11, ist die Mutter 14 angeordnet. Daraus wird deutlich, daß der als Zylinderstück 15 ausgebildete Aufnahmehohlkörper, welcher die im radialen Abstand und senkrecht zur Zylinderstück-Längsachsenebene angeordnete Bohrung 16 besitzt, axial verstellbar und in einer Endlage fixierbar ist. Wird nämlich an der Mutter 14 gedreht, dann bewegt sich das Zylinderstück 15 zur Rasterplatte hin, wobei die Rasten einerseits an der Rasterplatte, nämlich die Rasten 13 mit den Rasten 13a am Zylinderstück, in Eingriff kommen.

Die Bohrung 16 nimmt die Verlängerungsstange 17 auf. Die Befestigung der Verlängerungsstange 17 geschieht mit Hilfe einer Fixierschraube 18, die spitzwinklig zu der Tangentialbohrung 16 angeordnet ist. Durch diese Ausbildung des Aufnahmehohlkörpers als fixierbares Zylinderstück mit Tangentialbohrung ist die Handhabung des Gerätes einfacher, weil die einzusetzenden Teile und die festzustellenden Teile leicht zugänglich sind.

Hinzukommt, daß durch das Zwischengelenkstück 5 bzw. 5a und die Verstellbarkeit des Zylinderstücks auf dem Gewindezapfen 10 jede beliebige Lage des Verlängerungsstückes einstellbar ist, wodurch auch die auf der Knochenschraube 19 aufgesetzten Verlängerungsstäbe 17, 17a entsprechend eingerichtet werden können, was eine entsprechende Einrichtung der in die gesunden Wirbelkörper eingeschraubten Knochenschrauben bewerkstelligt.

Auch die besondere Ausbildung der Verlängerungsstange und der dazugehörigen Knochenschraube stellen eine Verbesserung hinsichtlich der Handhabung des Gerätes durch den Operateur dar.

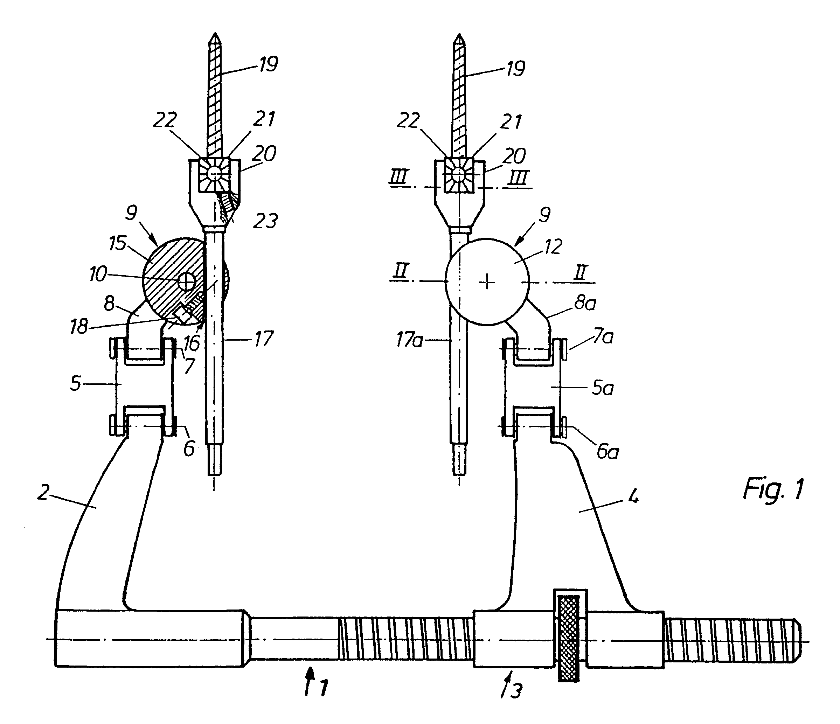

Dies wird dadurch erreicht, daß die Verlängerungsstange endseitig eine wannenförmige Ausbildung 20 aufweist, die von der Knochenschraube 19 aufgenommen wird. Dabei hat die Knochenschraube kopfseitig ein entsprechend gestaltetes Profilstück 21 mit einer an der Oberseite angeordneten Rasterung 22. Die Befestigung des Profilstückes 21 in der wannenförmigen Ausbildung 20 erfolgt ebenfalls mit einer Schraube 23, welche spitzwinklig zur Achse der Verlängerungsstange 17 verläuft (Fig. 1).

Die wannenförmige Ausbildung 20 weist dabei mehr als Halbkreisform auf, damit die Knochenschraube 19 mit ihrem Profilstück 21 längs eingeschoben werden kann bzw. längs herausgezogen werden kann (Fig. 3).

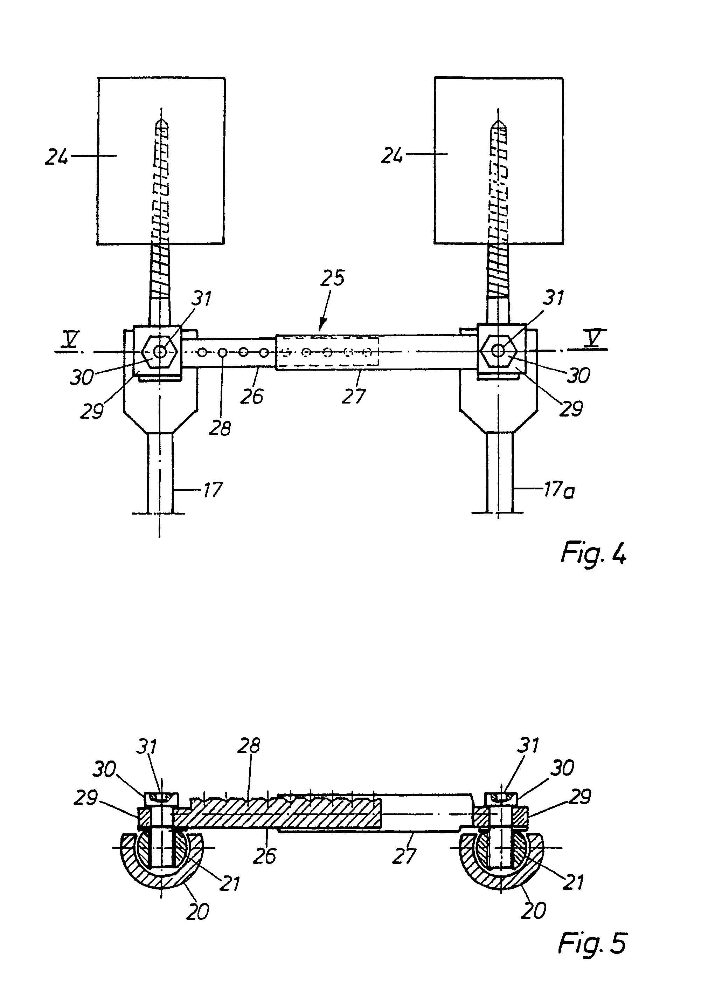

Sind die Wirbel 24 mit Hilfe dieser Vorrichtung eingerichtet, dann muß diese Lage fixiert werden. Das geschieht mit Hilfe eines Fixierstabes 25, der aus den Teleskopstücken 26 und 27 zusammengesetzt ist. Die Fixierung der beiden Teile geschieht dadurch, daß der einschiebbare Teil 26 Fixierkerben 28 besitzt. Dadurch kann das andere hülsenförmige Teleskopstück 27 mit dem einschiebbaren Teil dadurch fixiert werden, daß mit Hilfe einer besonderen Zange auf das hülsenförmige Teleskopstück im Bereich der Fixierkerben gedrückt wird, so daß ein Zusammenquetschen erfolgt. Dabei kann eine Lochschablone als Hilfsmittel dienen, die beispielsweise als U-Schienenstück ausgebildet ist und in gleichem Abstand Bohrungen aufweist, wie die Fixierkerben angebracht sind (Fig. 4 und 5).

Der Fixierstab weist endseitig eine mit Bohrung versehene Rasterplatte 29 auf, die mit der Rasterung 22 des Profilstückes 21 in Eingriff kommt, wenn beide Teile durch eine Schraube 30 verbunden werden (vergl. Fig. 5).

Damit das Einsetzen der Schraube 30 erleichtert ist, besitzt die Schraube mittig eine Bohrung 31, in die ein Zapfen eigesetzt wird, welcher ein Griffstück besitzt (nicht dargestellt). Dadurch kann die sozusagen an dem Griffstück mit Hilfe des Zapfens sitzende Schraube leichter in das Gewinde eingeführt werden.

Ist der Fixierstab 25 an Ort und Stelle gebracht, dann ist die Operation abgeschlossen, so daß nunmehr die Vorrichtung zusammen mit den Verlängerungsstangen 17 bzw. 17a abgenommen werden kann.

Bei kurzen überbrückungslängen kann auch ein einstückiger Fixierstab mit endseitigen Rasterplatten mit Bohrung verwendet werden. Ist ein Verstellen der normalerweise in einer Ebene liegenden Rasterplatten erforderlich, dann kann das durch einfaches Verdrehen der Rasterplatte um die Längsachse des Fixierstabes geschehen.

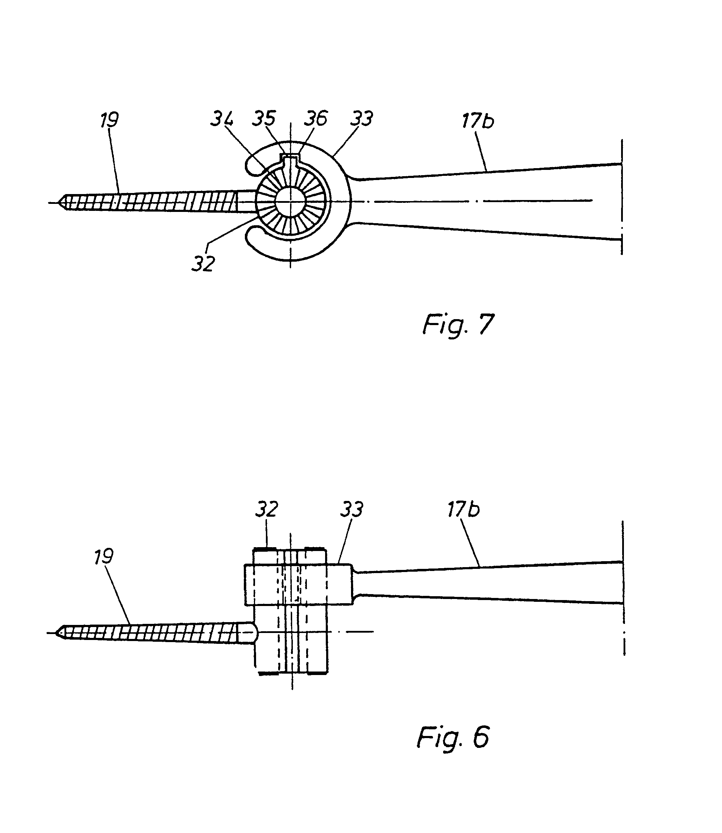

Eine andere Ausbildung der Verlängerungsstange bzw. Knochenschraube ist in Fig. 6 und 7 dargestellt. Dabei besitzt die Knochenschraube 19 ein zylinderförmiges Walzenstück 32, auf das ein entsprechend ausgebildetes Gabelstück 33 aufschiebbar ist, an das sich die Verlängerungsstange 17b anschließt. Die Stirnseite des Walzenstückes 32 besitzt Rasterungen 34 zur Aufnahme des Fixierstabes, wie bereits beschrieben. Damit das Walzenstück 32 undrehbar im Gabelstück 33 lagert, ist ein Ansatz 35 am Walzenstück angebracht, der in eine entsprechende Aussparung 36 im Gabelstück eingreift.

## Patentansprüche

1. Vorrichtung zum Einrichten einer Wirbelsäule mit geschädigten Wirbelkörpern, bestehend aus einem auf einer Führungsachse (1) verstellbarem Arm (4) und einem darauf angeordneten festen Arm (2), wobei jeder Arm (2, 4) einen in zwei Ebenen verstellbaren Aufnahmehohlkörper (9) für eine Verlängerungsstange (17, 17a, 17b) trägt, die mit einer Knochenschraube (19) verbindbar ist, **dadurch gekennzeichnet**, daß jeder Arm (2, 4) ein Zwischengelenkstück (5, 5a) mit je zwei Gelenken (6, 7) trägt, wobei am Zwischengelenkstück (5, 5a) ein Träger (8) für den Aufnahmeholkörper angelenkt ist, der als fixierbares Zylinderstück (15) mit einer Bohrung (16) ausgebildet ist, die im radialen Abstand und senkrecht zur Zylinderstück-Längsachsenebene angeordnet ist.

2. Vorrichtung nach Anspruch 1 **dadurch gekennzeichnet**, daß das Zylinderstück (15) axial verstellbar und in einer Endlage feststellbar ist.

3. Vorrichtung nach Anspruch 1 **dadurch gekennzeichnet**, daß spitzwinklig zur Tangentialbohrung (16) eine Fixierschraube (18) zur Fixierung des Verlängerungsstabes (17, 17a) vorgesehen ist.

4. Vorrichtung nach Anspruch 1 und 2 **dadurch gekennzeichnet**, daß der Träger (8) einen senkrechten Gewindezapfen (10) trägt, der eine Mutter (14) und eine ortsfeste Rasterplatte (12) aufweist, wobei zwischen Mutter (14) und Rasterplatte (12) der Aufnahmehohlkörper drehbar und axial verstellbar angebracht ist.

5. Vorrichtung nach Anspruch 1 **dadurch gekennzeichnet**, daß die Verlängerungsstange (17, 17a) endseitig eine wannenförmige Ausbildung (20) für die Aufnahme der Knochenschraube (19) aufweist, die kopfseitig ein entsprechend gestaltetes Profilstück (21) mit Rasterung (22) für die Aufnahme eines Fixierstabes (25) aufweist.

6. Vorrichtung nach Anspruch 4 **dadurch gekennzeichnet** , daß die wannenförmige Ausbildung (20) mehr als Halbkreisform aufweist, so daß die Verlängerungsstange nur axial herausziehbar ist.

7. Vorrichtung nach Anspruch 4 **dadurch gekennzeichnet**, daß die Befestigung der Knochenschraube (19) in der wannenförmigen Ausbildung (20) der Verlängerungsstange (17, 17a) durch eine Schraube (23) erfolgt, die spitzwinklig zur Achse der Verlängerungsstange einschraubbar ist.

8. Vorrichtung nach Anspruch 1 **dadurch gekennzeichnet** , daß die Knochenschraube ein zylinderförmiges Walzenstück (32) aufweist, das stirnseitig eine Rasterung (34) für die Aufnahme eines Fixierstabes aufweist, und daß die Verlängerungsstange endseitig ein der Zylinderform entsprechendes Gabelstück (33) aufweist wobei die Verlängerungsstange auf dem Walzenstück drehfest angebracht ist.

9. Vorrichtung nach Anspruch 5 **dadurch gekennzeichnet**, daß der Fixierstab (25) aus Telekopstücken (26, 27) besteht, dessen Einschiebteil (26) Fixierkerben (28) aufweist.

10. Vorrichtung nach Anspruch 5 **dadurch gekennzeichnet**, daß der Fixierstab einstückig ausgebildet ist.

11. Vorrichtung nach Anspruch 9 und 10 **dadurch gekennzeichnet**, daß der Fixierstab endseitig eine Rasterplatte (29) mit Bohrung aufweist.

12. Vorrichtung nach Anspruch 5 **dadurch gekennzeichnet**, daß die Verbindung des Fixlerstabes mit der Knochenschraube durch eine Schraube (30) erfolgt, die mittig eine Vertiefung (31) für den Einsatz eines Haltestiftes mit Griff aufweist.

## Claims

1. Device for setting a spinal column with damaged vertebrae and consisting of an arm (4), which is adjustable on a guide axle (1), and a fixed arm (2) arranged thereon, wherein each arm (2, 4) carries a hollow receiving body (9), which is adjustable in two planes, for an extension rod (17, 17a, 17b), which is connectable with a bone screw (19), characterised thereby, that each arm (2, 4) carries a respective intermediate hinge member (5, 5a) each with two hinges (6, 7), wherein a carrier (8) for the hollow receiving body (9), which is constructed as fixable cylindrical member (15) with a bore (16), which is arranged at a radial spacing from and perpendicularly to the longitudinal axial plane of the cylindrical member, is articulated at the intermediate hinge member (5, 5a).

2. Device according to claim 1, characterised thereby, that the cylindrical member (15) is adjustable axially and fixable in an end position.

3. Device according to claim 1, characterised thereby, that a fixing screw (18) for fixing the extension rod (17, 17a) is provided at an acute angle to the tangential bore (16).

4. Device according to claim 1 and 2, characterised thereby, that the carrier (8) carries a perpendicular threaded spigot (10), which displays a nut (14) and a fixedly located raster plate (12), wherein the hollow receiving body is mounted to be rotatable and axially displaceable between the nut (14) and the raster plate (12).

5. Device according to claim 1, characterised thereby, that the extension rod (17, 17a) at the end displays a trough-shaped formation (20) for the reception of the bone screw (19), which at the head end displays a correspondingly structured profile member (21) with rastering (22) for the reception of a fixing rod (25).

6. Device according to claim 4, characterised thereby, that the trough-shaped formation (20) displays more than semicircular shape so that the extension rod is withdrawable only axially.

7. Device according to claim 4, characterised thereby, that the fastening of the bone screw (19) in the trough-shaped formation (20) of the extension rod (17, 17a) takes place by a screw (23), which is able to be screwed in at an acute angle to the axis of the extension rod.

8. Device according to claim 1, characterised thereby, that the bone screw displays a cylindrical roll member (32), which at an end face displays a rastering (34) for the reception of a fixing rod and that the extension rod at the end displays a fork member (33) corresponding with the cylindrical shape, wherein the extension rod is mounted on the roll member to be secure against rotation.

9. Device according to claim 5, characterised thereby, that the fixing rod (25) consists of telescopic members (26, 27), the insert part (26) displays fixing notches (28).

10. Device according to claim 5, characterised thereby, that the fixing rod is constructed in one piece.

11. Device according to claim 9 and 10, characterised thereby, that the fixing rod at one end displays a raster plate (29) with bore.

12. Device according to claim 5, characterised thereby, that the connection of the fixing rod with the bone screw takes place through a screw (30), which centrally displays a depression (31) for the insertion of a retaining pin with handle.

## Revendications

1. Dispositif pour ajuster une colonne vertébrale dont des vertèbres sont endommagées, comportant un bras (4) réglable sur un axe de guidage (1) et un bras fixe (2) monté sur cet axe, chaque bras (2, 4) portant une monture creuse (9) ajustable dans deux plans et destinée à recevoir une tige de rallonge (17, 17a, 17b) agencée pour être fixée à une vis à os (19), caractérisé en ce que chaque bras (2, 4) porte une pièce intermédiaire (5, 5a) pourvue de deux articulations (6, 7), cette pièce intermédiaire (5, 5a) étant articulée à un support (8) portant la monture creuse, laquelle est formée par une pièce cylindrique (15) pouvant être fixée et ayant un alésage (16) disposé à distance radiale et perpendiculairement à l'axe longitudinal de la pièce cylindrique.

2. Dispositif selon la revendication 1, caractérisé en ce que la pièce cylindrique (15) est ajustable axialement et peut être fixée dans une position terminale.

3. Dispositif selon la revendication 1, caractérisé en ce qu'une vis de fixation (18) est disposée à angle aigu par rapport à l'alésage tangentiel (16) pour fixer la tige de rallonge (17, 17a).

4. Dispositif selon les revendications 1 et 2, caractérisé en ce que le support (8) porte un téton fileté perpendiculaire (10) pourvu d'un écrou (14) et d'une plaque crantée fixe (12), la monture creuse étant montée de manière réglable en rotation et axialement entre l'écrou (14) et la plaque crantée (12).

5. Dispositif selon la revendication 1, caractérisé en ce que la tige de rallonge (17, 17a) présente, à une extrémité, une forme concave (20) destinée à recevoir la vis à os (19), laquelle comporte une tête profilée correspondante (21) pourvue de crans (22) pour la mise en place d'une barre de fixation (25).

6. Dispositif selon la revendication 4, caractérisé en ce que ladite forme concave (20) couvre plus d'un demi-cercle, de sorte que la tige de rallonge ne peut être extraite qu'axialement.

7. Dispositif selon la revendication 4, caractérisé en ce que la fixation de la vis à os (19) dans ladite forme concave (20) de la tige de rallonge (17, 17a) est faite au moyen d'une vis (23) agencée pour être vissée sous un angle aigu par rapport à l'axe de la tige de rallonge.

8. Dispositif selon la revendication 1, caractérisé en ce que la vis à os comporte une tête à forme cylindrique (32) ayant, sur une face frontale, des crans (34) pour la mise en place d'une barre de fixation, et en ce que la tige de rallonge présente, à une extrémité, une fourche (33) correspondant à ladite forme cylindrique et grâce à laquelle la tige de rallonge est montée de manière fixe en rotation sur ladite tête

9. Dispositif selon la revendication 5, caractérisé en ce que la barre de fixation (25) est formée d'éléments télescopiques (26, 27) dont la partie s'emboîtant (26) comporte des encoches de fixation (28).

10. Dispositif selon la revendication 5, caractérisé en ce que la barre de fixation est faite d'une seule pièce.

11. Dispositif selon les revendications 9 et 10, caractérisé en ce que la barre de fixation comporte à une extrémité une plaque crantée (29) pourvue d'un alésage.

12. Dispositif selon la revendication 5, caractérisé en ce que l'assemblage entre la barre de fixation et la vis à os s'effectue au moyen d'une vis (30) comportant en son centre une cavité (31) pour l'introduction d'une goupille de serrage pourvue d'une poignée.

Fig. 1

EP 0 316 371 B1

Fig. 2

Fig. 3

*Fig. 4*

*Fig. 5*

Fig. 7

Fig. 6